(19) Europäisches Patentamt
European Patent Office
Office européen des brevets

(11) EP 1 312 345 B1

(12) FASCICULE DE BREVET EUROPEEN

(45) Date de publication et mention de la délivrance du brevet:
**07.04.2010 Bulletin 2010/14**

(51) Int Cl.:
***A61K 8/898*** *(2006.01)* ***A61Q 5/00*** *(2006.01)*

(21) Numéro de dépôt: **02292746.1**

(22) Date de dépôt: **04.11.2002**

(54) **Utilisation de silicones aminées particulières en pré-traitement de colorations directes ou d'oxydation de fibres kératiniques**

Verwendung von speziellen Aminosilikonen in der Vorbehandlung zur direkten oder oxydativen Färbung von Keratinfasern

Use of aminosilicones as a pre-treatment in direct or oxydation dyeing of keratinous fibres

(84) Etats contractants désignés:
**AT BE BG CH CY CZ DE DK EE ES FI FR GB GR IE IT LI LU MC NL PT SE SK TR**

(30) Priorité: **08.11.2001 FR 0114483**

(43) Date de publication de la demande:
**21.05.2003 Bulletin 2003/21**

(73) Titulaire: **L'Oréal**
**75008 Paris (FR)**

(72) Inventeurs:
- **Devin-Baudoin, Priscille**
**92170 Vanves (FR)**
- **Sabbagh, Anne**
**92500 Rueil Malmaison (FR)**

(74) Mandataire: **Fevrier, Murielle Françoise E.**
**L'Oréal**
**D.I.P.I.**
**25-29 Quai Aulagnier**
**92600 Asnières (FR)**

(56) Documents cités:
**EP-A- 0 890 355** **DE-A- 19 754 053**
**GB-A- 2 165 550**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 312 345 B1

Printed by Jouve, 75001 PARIS (FR)

## Description

La présente invention a pour objet l'utilisation, en pré-traitement d'une coloration directe ou d'oxydation des fibres kératiniques humaines et plus particulièrement des cheveux, d'une composition comprenant au moins une silicone aminée particulière.
Elle a aussi pour objet un procédé de coloration directe ou d'oxydation des fibres kératiniques humaines et plus particulièrement des cheveux comprenant un pré-traitement avec une composition comprenant au moins une silicone aminée particulière.

Il existe principalement deux types de coloration des fibres kératiniques. La coloration directe, mettant en oeuvre en présence ou en l'absence d'agents oxydants, des colorants directs et / ou des pigments qui sont des molécules colorées, conférant aux fibres une couleur temporaire qui s'estompe après quelques shampooings, et la coloration dite "coloration d'oxydation" mettant en oeuvre des précurseurs de colorants d'oxydation et un agent oxydant, qui confère aux fibres une couleur plus tenace que la précédente.

Le document EP 890335 décrit une composition cosmétique pour la coloration d'oxydation des cheveux comprenant une silicone aminée qui peut être substituée par un radical alcoxy.

Il existe un besoin d'améliorer la montée de ces colorations sur les fibres, en particulier sur des fibres sensibilisées car celles-ci sont plus poreuses et fixent moins les colorants.
Par ailleurs, l'utilisation d'un agent oxydant entraîne en général une certaine dégradation de la fibre kératinique.
Il existe donc un besoin de limiter ces dégradations et les conséquences qu'elles entraînent sur l'état cosmétique de la fibre.

Après d'importantes études sur la question, la demanderesse a découvert de manière tout à fait inattendue et surprenante que l'utilisation, en pré-traitement sur des fibres kératiniques humaines et plus particulièrement des cheveux, d'une composition comprenant au moins une silicone aminée particulière, permettait de résoudre ces problèmes.
Cette découverte est à l'origine de la présente invention.

En outre, ce pré-traitement améliore la ténacité des colorations directes ou d'oxydation, en particulier vis à vis des shampooings.

Un premier objet de l'invention concerne donc l'utilisation, en pré-traitement d'une coloration d'oxydation ou d'une coloration directe des fibres kératiniques humaines et plus particulièrement des cheveux, d'une composition comprenant au moins une silicone aminée de formules (I) ou (II) décrites ci-après.

Ladite utilisation a notamment pour objet d'améliorer la montée de la couleur, en particulier sur des cheveux sensibilisés et/ou l'état de la fibre après coloration, notamment dans le cas de coloration avec oxydant ainsi que la ténacité aux shampooings desdites colorations.

Par amélioration de l'état de la fibre on entend une diminution de la porosité ou de la solubilité alcaline de la fibre et une amélioration des propriétés cosmétiques et en particulier du lissage, de la douceur et de la facilité de démêlage et de coiffage.
Cet effet est rémanent, c'est à dire durable.
La porosité se mesure par la fixation à 37°C et à pH10, en 2 minutes, de la 2-nitro paraphénylènediamine à 0,25% dans un mélange éthanol / tampon pH 10 (rapport volumique 10/90).
La solubilité alcaline correspond à la perte de masse d'un échantillon de 100 mg de fibres kératiniques sous l'action de la soude décinormale pendant 30 minutes à 65°C.

Un second objet de l'invention concerne un procédé de coloration consistant à appliquer sur les fibres kératiniques humaines et plus particulièrement des cheveux, dans une première étape, une composition comprenant au moins une silicone aminée de formules (I) ou (II), à rincer ou non rincer les fibres, puis dans une seconde étape, à appliquer une composition colorante directe ou d'oxydation pendant un temps suffisant pour développer la couleur, à faire suivre ou non par un rinçage, puis éventuellement un shampooing, et ensuite un séchage.

Silicones aminées

Les silicones aminées de formules (I) ou (II) selon l'invention sont les suivantes :

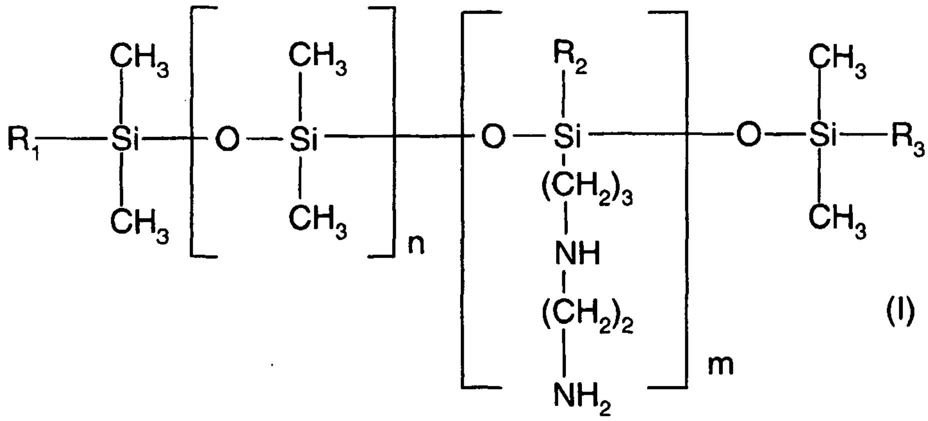

(I)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5;
$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence le radical alcoxy est un radical méthoxy.
Le rapport molaire Hydroxy/Alcoxy est de préférence compris entre 0,2:1 et 0,4:1 et de préférence entre 0,25:1 et 0,35: 1 et plus particulièrement est égal à 0,3.

La silicone aminée de formule (I) présente une masse moléculaire moyenne en poids allant de préférence de 2000 à 1000000 et encore plus particulièrement de 3500 à 200000.

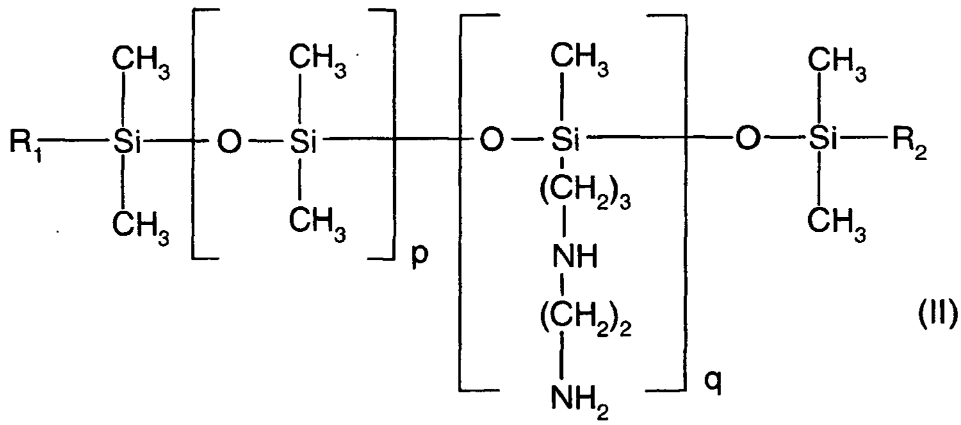

(II)

dans laquelle :

p et q sont des nombres tels que la somme (p + q) peut varier notamment de 1 à 1 000 et en particulier de 50 à 350, et plus particulièrement de 150 à 250
p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;
$R_1$, $R_2$, différents, représentent un radical hydroxy ou alcoxy en $C_1$-$C_4$, l'un au moins des radicaux $R_1$ à $R_2$ désignant un radical alcoxy.

De préférence le radical alcoxy est un radical méthoxy.

Le rapport molaire Hydroxy/Alcoxy est de préférence compris entre 1:0,8 et 1:1,1 et de préférence entre 1:0,9 et 1:1 et plus particulièrement est égal à 1:0,95.

La silicone aminée de formule (II) présente une masse moléculaire moyenne en poids allant de préférence de 2000 à 200.000, et encore plus particulièrement de 5.000 à 100.000 et plus particulièrement encore de 10.000 à 50.000.

Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.

Les produits commerciaux correspondant à ces silicones de structure (I) ou (II) peuvent inclure dans leur composition une ou plusieurs autres silicones aminées dont la structure est différente des structures (I) et (II).

Un produit contenant des silicones aminées de structure (I) est proposé par la société WACKER sous la dénomination BELSIL ADM 652®.

Un produit contenant des silicones aminées de structure (II) est proposé par la société WACKER sous la dénomination Fluid WR 1300®.

Lorsque ces silicones aminées sont mises en oeuvre, une forme de réalisation particulièrement intéressante est leur utilisation sous forme d'émulsion huile-dans-eau. L'émulsion huile-dans-eau peut comprendre un ou plusieurs tensioactifs. Les tensioactifs peuvent être de toute nature mais de préférence cationique et/ou non ionique.

Les particules de silicone dans l'émulsion ont une taille moyenne allant généralement de 3 nm à 500 nanomètres. De préférence, notamment pour les silicones aminées de formule (II), on utilise des microémulsions de taille allant de 5 nm à 60 nanomètres et plus particulièrement de 10 nm à 50 nanomètres.

On peut utiliser selon l'invention les microémulsions de silicones aminées de formule (II) proposées sous la dénomination FINISH CT 96 E® ou SLM 28020® par la société WACKER.

De préférence la silicone aminée de formule (I) ou (II) est choisie de telle façon que l'angle de contact avec l'eau d'un cheveu traité avec une composition contenant 2% MA (matières actives) de ladite silicone selon l'invention soit compris entre 90 et 180° et de préférence entre 90 et 130°, bornes incluses.

De préférence la composition contenant la ou les silicones aminées de formule (I) ou (II) est telle que l'angle de contact d'un cheveu traité avec la dite composition est compris entre 90 et 180° et de préférence entre 90 et 130°, bornes incluses.

La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90°, hydrophobe lorsque cet angle est compris entre 90° et 180°.

Le test s'effectue avec des mèches de cheveux naturels ayant été décolorés dans les mêmes conditions puis lavés. Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée pendant 30 secondes. La mèche essorée est laissée à l'air libre jusqu'à ce qu'elle soit complètement sèche.

Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.

Parallèlement, le périmètre du cheveu (P) est mesuré *via* une observation microscopique.

La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, $\Gamma lv$ la tension interfaciale liquide / vapeur de l'eau en $J/m^2$ et θ l'angle de contact.

Le produit SLM 28020® de WACKER à 12% dans l'eau (soit 2% en matières actives) conduit à un angle de contact de 93° selon le test indiqué ci-dessus.

La silicone aminée est utilisée de préférence dans la composition de pré-traitement en une quantité allant de 0,01 à 20% en poids du poids total de la composition. Plus préférentiellement, cette quantité va de 0,1 à 15% en poids et encore plus particulièrement de 0,5 à 10 % en poids.

La composition de pré-traitement peut contenir tous les ingrédients classiquement utilisés en cosmétique et en particulier dans le domaine capillaire. En particulier elle peut contenir des tensioactifs et/ou des polymères additionnels.

Ces tensioactifs et ces polymères peuvent être de nature non-ionique, cationique, anionique ou amphotère.
Parmi les polymères additionnels, les silicones aminées autres que celles de l'invention sont particulièrement préférées.
La composition de pré-traitement présente un pH allant de 2 à 11 et de préférence de 4 à 9.
Elle peut se présenter sous diverses formes telles que lotions, gels, crèmes, shampooings, sticks, mousses, sprays. Pour certaines de ces formes, elle peut être conditionnée en flacon pompe ou dans un récipient aérosol. Dans le cas de l'aérosol, la composition est associée à un agent propulseur qui peut être par exemple un alcane ou un mélange d'alcane, du diméthyl éther, de l'azote, du protoxyde d'azote, du gaz carbonique ou des halogénoalcanes, ainsi que leurs mélanges.
Une forme particulièrement préférée selon l'invention est la forme shampooing.
Dans ce cas, la composition contient au moins un agent tensioactif qui est de préférence anionique. De préférence alors, elle contient un mélange de tensioactifs dont au moins un agent tensioactif anionique, le ou les autres tensioactifs étant préférentiellement non ioniques ou amphotères.
La composition de pré-traitement peut être utilisée en mode rincé ou en mode non-rincé, c'est à dire que son application est suivie ou non d'un rinçage.
Dans le premier cas, le temps de pose de la composition de pré-traitement est compris entre quelques secondes et 60 minutes et de préférence entre 30 secondes et 15 minutes.
La température d'application de la composition de pré-traitement peut varier de 10°C à 70°C. De préférence l'application s'effectuera entre 10 et 60°C et plus particulièrement à la température ambiante.
La nature et la concentration des colorants présents dans les compositions colorantes n'est pas critique.
Dans le cas des colorations directes ( en présence ou en l'absence d'agents oxydants) les compositions colorantes comprennent au moins un colorant choisi parmi les colorants directs nitrés benzéniques neutres, acides ou cationiques, les colorants directs azoïques
ou méthiniques neutres, acides ou cationiques, les colorants directs quinoniques et en particulier anthraquinoniques neutres, acides ou cationiques, les colorants directs aziniques, les colorants directs triarylméthaniques, les colorants directs indoaminiques, les colorants directs naturels ou leurs mélanges.
Dans le cas des colorations d'oxydation, les compositions colorantes comprennent au moins une base d'oxydation.
Les bases d'oxydation sont choisies parmi celles classiquement utilisées en teinture d'oxydation, et parmi lesquelles on peut notamment citer les ortho- et paraphénylènediamines, les bases doubles, les ortho- et para- aminophénols, les bases hétérocycliques ainsi que leurs sels d'addition avec un acide .
Généralement, les compositions colorantes d'oxydation comprennent un ou plusieurs coupleurs.
Les coupleurs utilisables sont ceux classiquement utilisés dans les compositions de teinture d'oxydation, c'est-à-dire des métaphénylènediamines, des métaaminophénols et des métadiphénols, les dérivés mono- ou poly-hydroxylés du naphtalène, le sésamol et ses dérivés et des composés hétérocycliques tels que par exemple les coupleurs indoliques, les coupleurs indoliniques, les coupleurs pyridiniques et leurs sels d'addition avec un acide.
La nature de l'agent oxydant utilisé dans la coloration directe éclaircissante (coloration directe avec un agent oxydant) ou dans la coloration d'oxydation n'est pas critique.
L'agent oxydant est de préférence choisi dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.
Les exemples suivants sont destinés à illustrer l'invention. Celle-ci n'est cependant pas limitée à ces modes de réalisation.

<u>EXEMPLES</u>

On a préparé les 3 compositions de pré-traitement A, B, C, suivantes.
[exprimées en grammes de Matière Active]

| **Composition A** | |
|---|---|
| Polydiméthylsiloxane de formule (II) selon l'invention : SLM 28020/2® de la société WACKER | 2 |
| Eau déminéralisée q.s.p | 100 |

| Composition B | |
|---|---|
| Polydiméthylsiloxane de formule (II) selon l'invention : FINISH CT 96 E® de la société WACKER | 2 |
| Eau déminéralisée q.s.p | 100 |

| Composition C | |
|---|---|
| Polydiméthylsiloxane de formule (II) selon l'invention : FINISH WR 1300® de la société WACKER | 2 |
| Alcool cétylstéarylique/lauryl sulfate de sodium/myristate de cétyle/ alcool myristique (62/20/8/10) | 12 |
| Glycérine | 0,5 |
| Alcool oléïque oxyéthyléné (20 OE) | 0,1 |
| Eau déminéralisée q.s.p | 100 |

La composition A a été appliquée sur une mèche de cheveux gris à 90% de blancs.
Après 5 minutes de pose, et sans rinçage intermédiaire, on a coloré la mèche avec une coloration d'oxydation du commerce MAJIREL® de la société L'OREAL.
On a évalué ensuite la porosité des cheveux et leur solubilité alcaline via les méthodes précédemment décrites. On a obtenu les résultats suivants :

Coloration MAJIREL® :
Porosité = 24 +/-1
Solubilité alcaline : 9,4 +/- 0,5
Pré-traitement suivi de coloration MAJIREL® :
Porosité : 19 +/-4
Solubilité alcaline : 5,8 +/- 0,5
Témoin : cheveux non colorés non traités :
Porosité : 17 +/- 1
Solubilité alcaline : 6,3 +/- 0,7.

Les cheveux ayant subi un pré-traitement selon l'invention ont donc été moins dégradés.
La composition B a été appliquée sur la chevelure de 8 modèles.
Sans rinçage, on a ensuite appliqué une coloration d'oxydation du commerce KARIZMA CREME COLOUR® de la société SOFT SHEEN.
On a constaté qu'après le traitement, les cheveux étaient doux, légers et faciles à démêler.
Les résultats ont été supérieurs à ceux que, comparativement, on a obtenus en utilisant le pré-traitement avant coloration de la gamme KARIZMA CREME COLOUR, pré-traitement du commerce qui ne contient pas de silicone aminée de formule (I) ou (II) selon l'invention.
La composition B a également été appliquée pendant 15 minutes à 60°C sur des cheveux moyennement décolorés. Les cheveux ont ensuite été rincés.
On a alors appliqué sur ces cheveux :

- d'une part, une coloration directe EXPRESSION® nuance cuivrée de la société L'OREAL (pose 15 minutes) ;
- d'autre part, un mélange de paraphénylènediamine à 3. $10^{-3}$ moles/100 g et de dichlorhydrate de 2,4-diaminophénoxyéthanol à 3. $10^{-3}$ moles/100 g dans une base classique de coloration d'oxydation du commerce RECITAL® de la société L'OREAL que l'on a mélangé poids pour poids avec de l'eau oxygénée à 20 volumes avant l'application (pose du mélange 30 minutes).

On a évalué alors l'intensité de montée via le paramètre colorimétrique L* du système L*a*b*. On a obtenu les résultats suivants :

EXPRESSION® : Pré-traitement avec silicone : L*=38
Pré-traitement avec eau : L*=40
RECITAL® : Pré-traitement avec silicone : L*=16
Pré-traitement avec eau : L*=20

Ces résultats, significatifs, ont indiqué une meilleure montée avec le pré-traitement de l'invention, (l'intensité est d'autant plus forte que L* est faible).

La composition C a été appliquée pendant 10 minutes sur des cheveux naturels, puis elle a été rincée. On a appliqué ensuite une coloration d'oxydation classique du commerce. En final, l'état des cheveux a été satisfaisant et les propriétés cosmétiques ont été bonnes (douceur et lissage).

## Revendications

1. Utilisation en pré-traitement d'une coloration directe ou d'oxydation des fibres kératiniques humaines, d'une composition comprenant au moins une silicone aminée de formules (I) ou (II) suivantes :

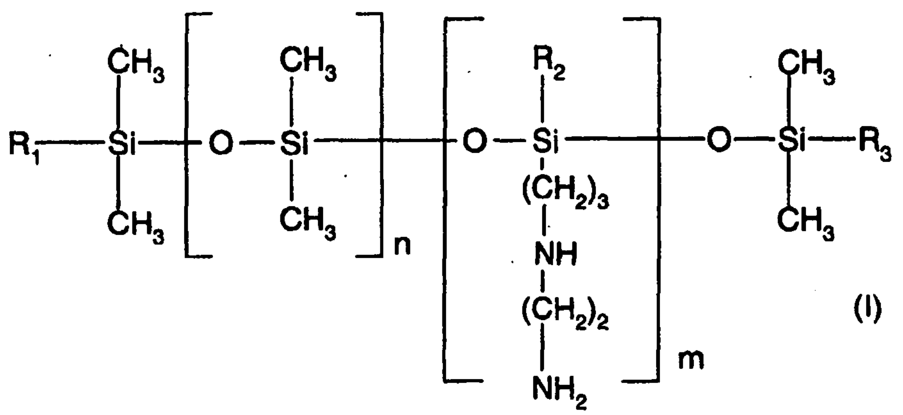

(I)

formule (I) dans laquelle :
m et n sont des nombres tels que la somme (n + m) varie de 1 à 1 000,
n désignant un nombre de 0 à 999 et m désignant un nombre de 1 à 1 000,
$R_1$, $R_2$, $R_3$, identiques ou différents, représentent un radical hydroxy ou alcoxy en $C_1$-$C_4$ ; l'un au moins des radicaux $R_1$ à $R_3$ désignant un radical alcoxy ;

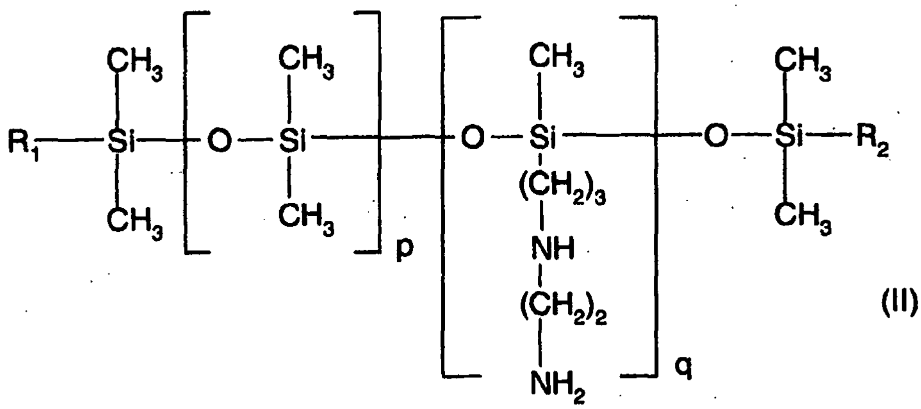

(II)

formule (II) dans laquelle :
p et q sont des nombres tels que la somme (p + q) varie de 1 à 1 000,
p désignant un nombre de 0 à 999 et q désignant un nombre de 1 à 1 000.
$R_1$, $R_2$, différents, représentent un radical hydroxy ou alcoxy en $C_1$-$C_4$, l'un au moins des radicaux $R_1$ à $R_2$ désignant un radical alcoxy.

2. Utilisation selon la revendication 1, **caractérisée par le fait que** le radical alcoxy $C_1$-$C_4$ désigne le radical méthoxy.

3. Utilisation selon l'une quelconque des revendications précédentes, **caractérisée par le fait que** pour les silicones aminées de formule (I), le rapport molaire Hydroxy/Alcoxy est compris entre 0,2:1 et 0,4:1.

4. Utilisation selon l'une quelconque des revendications 1 à 2, **caractérisée par** le fait pour que les silicones aminées de formule (II), le rapport molaire Hydroxy/Alcoxy est compris entre 1:0,8 et 1:1,1.

5. Utilisation selon l'une quelconque des revendications 1 à 3, **caractérisée par le fait que** la silicone aminée de formule (I) présente une masse moléculaire moyenne en poids allant de 2000 à 1000000.

6. Utilisation selon l'une quelconque des revendications 1 à 2 et 4, **caractérisée par le fait que** la silicone aminée de formule (II) présente une masse moléculaire moyenne en poids allant de 2000 à 200.000.

7. Utilisation selon l'une quelconque des revendications précédentes, **caractérisée par le fait que** la silicone aminée de formule (I) ou (II) est sous forme d'émulsion huile-dans-eau comprenant des agents tensioactifs.

8. Utilisation selon la revendication 7, **caractérisée par le fait que** l'émulsion comprend au moins un agent tensioactif cationique et/ou non ionique.

9. Utilisation selon l'une quelconque des revendications 7 ou 8, **caractérisée par le fait que** les particules de silicone dans l'émulsion ont une taille allant de 3 nm à 500 nanomètres.

10. Utilisation selon l'une quelconque des revendications précédentes, **caractérisée par le fait que** la ou les silicones aminées de formules (I) ou (II) sont présentes dans la composition de pré-traitement en une quantité allant de 0,01 à 20% en poids du poids total de la composition.

11. Utilisation selon la revendication 10, **caractérisée par le fait que** la ou les silicones aminées de formule (I) ou (II) sont présentes dans la composition de pré-traitement en une quantité allant de 0,1 à 15% en poids du poids total de la composition.

12. Utilisation selon la revendication 11, **caractérisée par le fait que** la ou les silicones aminées de formule (I) ou (II) sont présentes dans la composition de pré-traitement en une quantité allant de 0,5 à 10 % en poids du poids total de la composition.

13. Utilisation selon l'une quelconque des revendications précédentes, **caractérisée par le fait que** la composition de pré-traitement se présente sous forme de lotions, gels, crèmes, shampooings, sticks, mousses, sprays.

14. Utilisation selon l'une quelconque des revendications précédentes, **caractérisée par le fait que** la composition de pré-traitement est conditionnée en flacon pompe ou dans un récipient aérosol.

15. Utilisation selon la revendication 14, **caractérisée par le fait que** la composition de pré-traitement comprend au moins un agent propulseur choisi dans le groupe formé par les alcanes, le diméthyl éther, l'azote, le protoxyde d'azote, le gaz carbonique ou les halogénoalcanes.

16. Utilisation selon l'une quelconque des revendications précédentes, **caractérisée par le fait que** la composition de pré-traitement comprend au moins un agent tensioactif de nature non-ionique, cationique, anionique ou amphotère.

17. Utilisation selon la revendication 16, **caractérisée par le fait que** la composition de pré-traitement comprend un mélange d'agents tensioactifs comprenant au moins un agent tensioactif anionique, le ou les autres agents tensioactifs étant non ioniques ou amphotères.

18. Utilisation selon l'une quelconque des revendications précédentes, **caractérisée par le fait que** la composition de pré-traitement comprend au moins un polymère additionnel autre que les silicones de formules (I) ou (II).

19. Utilisation selon la revendication 18, **caractérisée par le fait que** le polymère est de nature non-ionique, cationique, anionique ou amphotère.

**20.** Utilisation selon la revendication 19, **caractérisée par le fait que** le polymère est une silicone aminée différente des silicones de formules (I) ou (II).

**21.** Utilisation selon l'une quelconque des revendications précédentes, **caractérisée par le fait que** la composition de pré-traitement présente un pH allant de 2 à 11.

**22.** Utilisation selon la revendication 1 pour améliorer la montée de la coloration.

**23.** Utilisation selon la revendication 22 pour améliorer la montée de la coloration sur des cheveux sensibilisés.

**24.** Utilisation selon la revendication 23 pour améliorer l'état des fibres.

**25.** Utilisation selon la revendication 24 pour diminuer la porosité des fibres.

**26.** Utilisation selon la revendication 24 pour diminuer la solubilité alcaline des fibres.

**27.** Utilisation selon la revendication 1 pour améliorer la ténacité de la coloration aux shampooings.

**28.** Procédé de coloration des fibres kératiniques humaines
**caractérisé par le fait qu'**il consiste à appliquer sur les fibres, dans une première étape, une composition de prétraitement comprenant au moins une silicone de formule (I) ou (II) et telle que décrite à l'une quelconque des revendications 1 à 9, puis, après avoir rincé ou non rincé les fibres, appliquer dans une seconde étape, une composition colorante directe, à la laisser agir pendant un temps suffisant pour développer la couleur, puis rincer ou non rincer les fibres et les sécher.

**29.** Procédé de coloration des fibres kératiniques humaines
**caractérisé par le fait qu'**il consiste à appliquer sur les fibres, dans une première étape, une composition de prétraitement comprenant au moins une silicone de formule (I) ou (II) et telle que décrite à l'une quelconque des revendications 1 à 9, puis, après avoir rincé ou non rincé les fibres, appliquer dans une seconde étape, une composition colorante d'oxydation, à la laisser agir pendant un temps suffisant pour développer la couleur, puis rincer ou non rincer les fibres et les sécher.

**30.** Procédé selon l'une quelconque des revendications 28 ou 29, **caractérisé par le fait que** la composition de prétraitement est posée pendant un temps allant de quelques secondes à 60 minutes

**Claims**

**1.** Use, as a pretreatment of a process for colouring human keratin fibres with direct dyes or with oxidation dyes, of a composition comprising at least one aminosilicone of formula (I) or (II) below:

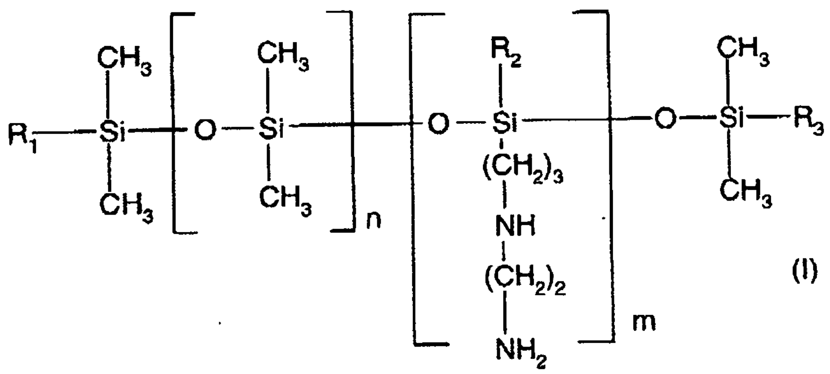

(I)

in which formula (I):
m and n are numbers such that the sum (n + m) ranges from 1 to 1000,

n denoting a number from 0 to 999 and m denoting a number from 1 to 1000;
$R_1$, $R_2$ and $R_3$, which may be identical or different, represent a hydroxyl or $C_1$-$C_4$ alkoxy radical, at least one of the radicals $R_1$ to $R_3$ denoting an alkoxy radical;

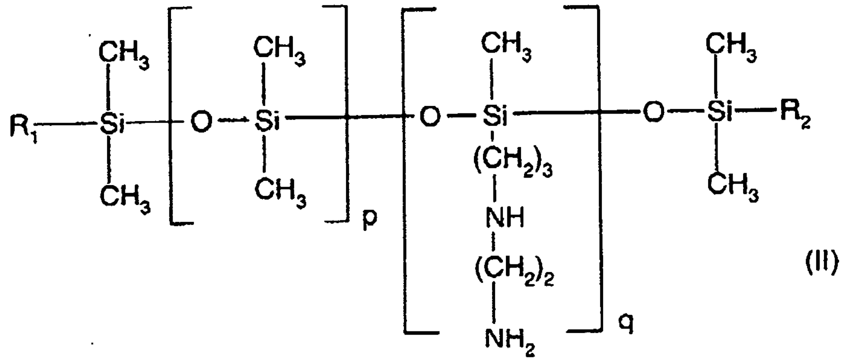

(II)

in which formula (II):
p and q are numbers such that the sum (p + q) ranges from 1 to 1000,
p denoting a number from 0 to 999 and q denoting a number from 1 to 1000;
$R_1$ and $R_2$, which are different, represent a hydroxyl or $C_1$-$C_4$ alkoxy radical, at least one of the radicals $R_1$ to $R_2$ denoting an alkoxy radical.

**2.** Use according to Claim 1, **characterized in that** the $C_1$-$C_4$ alkoxy radical denotes a methoxy radical.

**3.** Use according to either of the preceding claims, **characterized in that**, for the aminosilicones of formula (I), the hydroxyl/alkoxy molar ratio is between 0.2:1 and 0.4:1.

**4.** Use according to either of Claims 1 and 2,
**characterized in that**, for the aminosilicones of formula (II), the hydroxyl/alkoxy molar ratio is between 1:0.8 and 1:1.1.

**5.** Use according to any one of Claims 1 to 3,
**characterized in that** the aminosilicone of formula (I) has a weight-average molecular mass ranging from 2000 to 1 000 000.

**6.** Use according to any one of Claims 1, 2 and 4, **characterized in that** the aminosilicone of formula (II) has a weight-average molecular mass ranging from 2000 to 200 000.

**7.** Use according to any one of the preceding claims, **characterized in that** the aminosilicone of formula (I) or (II) is in the form of an oil-in-water emulsion comprising surfactants.

**8.** Use according to Claim 7, **characterized in that** the emulsion comprises at least one cationic and/or nonionic surfactant.

**9.** Use according to either of Claims 7 and 8,
**characterized in that** the silicone particles in the emulsion range in size from 3 nm to 500 nanometres.

**10.** Use according to any one of the preceding claims, **characterized in that** the aminosilicone(s) of formula (I) or (II) is (are) present in the pretreatment composition in an amount ranging from 0.01% to 20% by weight relative to the total weight of the composition.

**11.** Use according to Claim 10, **characterized in that** the aminosilicone(s) of formula (I) or (II) is (are) present in the pretreatment composition in an amount ranging from 0.1% to 15% by weight relative to the total weight of the composition.

**12.** Use according to Claim 11, **characterized in that** the aminosilicone(s) of formula (I) or (II) is (are) present in the

pretreatment composition in an amount ranging from 0.5% to 10% by weight relative to the total weight of the composition.

13. Use according to any one of the preceding claims, **characterized in that** the pretreatment composition is in the form of lotions, gels, creams, shampoos, sticks, mousses or sprays.

14. Use according to any one of the preceding claims, **characterized in that** the pretreatment composition is packaged in a pump-dispenser bottle or in an aerosol container.

15. Use according to Claim 14, **characterized in that** the pretreatment composition comprises at least one propellant chosen from the group formed by alkanes, dimethyl ether, nitrogen, nitrous oxide, carbon dioxide and haloalkanes.

16. Use according to any one of the preceding claims, **characterized in that** the pretreatment composition comprises at least one surfactant of nonionic, cationic, anionic or amphoteric nature.

17. Use according to Claim 16, **characterized in that** the pretreatment composition comprises a mixture of surfactants comprising at least one anionic surfactant, the other surfactant(s) being nonionic or amphoteric.

18. Use according to any one of the preceding claims, **characterized in that** the pretreatment composition comprises at least one additional polymer other than the silicones of formula (I) or (II).

19. Use according to Claim 18, **characterized in that** the polymer is of nonionic, cationic, anionic or amphoteric nature.

20. Use according to Claim 19, **characterized in that** the polymer is an aminosilicone different from the silicones of formula (I) or (II).

21. Use according to any one of the preceding claims, **characterized in that** the pretreatment composition has a pH ranging from 2 to 11.

22. Use according to Claim 1, for improving the rise of the coloration.

23. Use according to Claim 22, for improving the rise of the coloration on sensitized hair.

24. Use according to Claim 23, for improving the condition of the fibres.

25. Use according to Claim 24, for reducing the porosity of the fibres.

26. Use according to Claim 24, for reducing the alkaline solubility of the fibres.

27. Use according to Claim 1, for improving the resistance of the coloration with respect to shampooing.

28. Process for colouring human keratin fibres, **characterized in that** it consists in applying to the fibres, in a first step, a pretreatment composition comprising at least one silicone of formula (I) or (II) as described in any one of Claims 1 to 9, and then, after having optionally rinsed the fibres, in applying, in a second step, a direct dye composition, in leaving it to act for a time that is sufficient to develop the colour, and then in optionally rinsing the fibres, and drying them.

29. Process for colouring human keratin fibres, **characterized in that** it consists in applying to the fibres, in a first step, a pretreatment composition comprising at least one silicone of formula (I) or (II) as described in any one of Claims 1 to 9, and then, after having optionally rinsed the fibres, in applying, in a second step, an oxidation dye composition, in leaving it to act for a time that is sufficient to develop the colour, and then in optionally rinsing the fibres, and drying them.

30. Process according to either of Claims 28 and 29, **characterized in that** the pretreatment composition is left to act for a time ranging from a few seconds to 60 minutes.

**Patentansprüche**

1. Verwendung einer Zusammensetzung, die mindestens ein aminiertes Silicon der folgenden Formeln (I) oder (II) enthält, für die Vorbehandlung bei einer Direktfärbung oder oxidativen Färbung von menschlichen Keratinfasern:

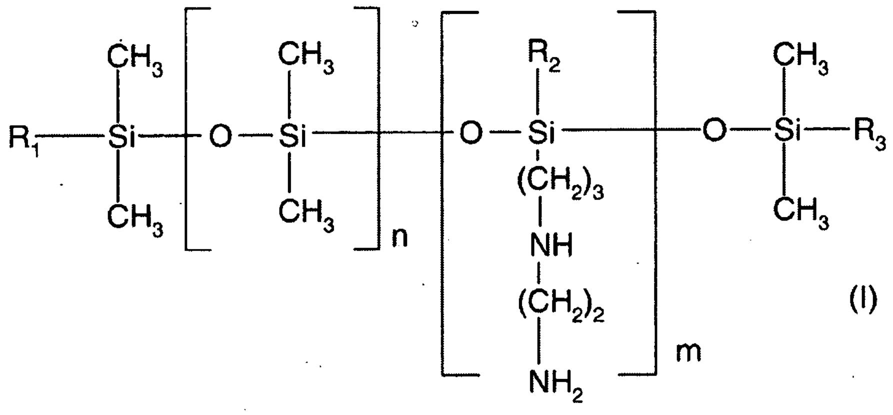

(I)

in der Formel (I):

m und n bedeuten Zahlen, die so gewählt sind, dass die Summe (n + m) im Bereich von 1 bis 1000 liegt, n ist eine Zahl im Bereich von 0 bis 999 und m ist eine Zahl im Bereich von 1 bis 1000, $R_1$, $R_2$ und $R_3$, die gleich oder verschieden sind, bedeuten eine Hydroxygruppe oder eine $C_{1-4}$-Alkoxygruppe, wobei mindestens eine der Gruppen $R_1$ bis $R_3$ eine Alkoxygruppe bedeutet;

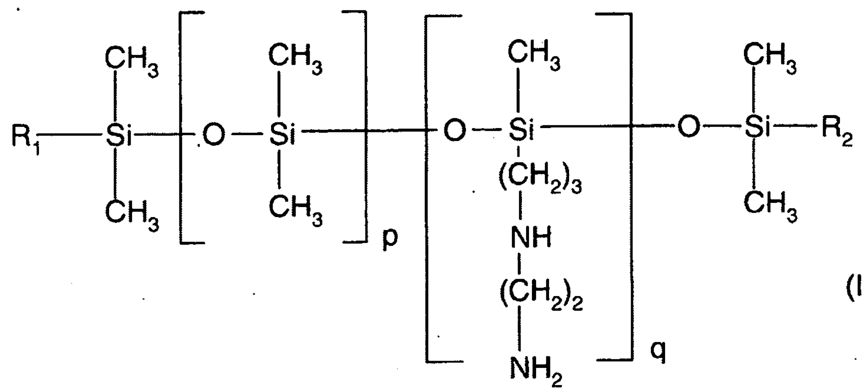

(II)

in der Formel (II):

p und q bedeuten Zahlen, die so gewählt sind, dass die Summe (p + q) im Bereich von 1 bis 1000 liegt, p ist eine Zahl im Bereich von 0 bis 999 und q ist eine Zahl im Bereich von 1 bis 1000, $R_1$ und $R_2$, die verschieden sind, bedeuten eine Hydroxygruppe oder eine $C_{1-4}$-Alkoxygruppe, wobei mindestens eine der Gruppen $R_1$ bis $R_2$ eine Alkoxygruppe bedeutet.

2. Verwendung nach Anspruch 1, **dadurch gekennzeichnet, dass** die $C_{1-4}$-Alkoxygruppe die Methoxygruppe bedeutet.

3. Verwendung nach einem der vorhergehenden Ansprüche, **dadurch gekennzeichnet, dass** für die aminierten Silicone der Formel (I) das Molverhältnis Hydroxy/Alkoxy im Bereich von 0,2:1 1 bis 0,4:1 liegt.

4. Verwendung nach einem der Ansprüche 1 bis 2, **dadurch gekennzeichnet, dass** für die aminierten Silicone der Formel (II) das Molverhältnis Hydroxy/Alkoxy im Bereich von 1:0,8 bis 1:1,1 liegt.

5. Verwendung nach einem der Ansprüche 1 bis 3, **dadurch gekennzeichnet, dass** das aminierte Silicon der Formel (I) eine gewichtmittlere Molmasse von 2.000 bis 1.000.000 aufweist.

6. Verwendung nach einem der Ansprüche 1 bis 2 und 4, **dadurch gekennzeichnet, dass** das aminierte Silicon der Formel (II) eine gewichtmittlere Molmasse von 2.000 bis 200.000 aufweist.

7. Verwendung nach einem der vorhergehenden Ansprüche, **dadurch gekennzeichnet, dass** das aminierte Silicon der Formel (I) oder (II) in Form einer Öl-in-Wasser-Emulsion vorliegt, die grenzflächenaktive Stoffe enthält.

8. Verwendung nach Anspruch 7, **dadurch gekennzeichnet, dass** die Emulsion mindestens einen kationischen und/oder nichtionischen grenzflächenaktiven Stoff enthält.

9. Verwendung nach einem der Ansprüche 7 oder 8, **dadurch gekennzeichnet, dass** die Siliconpartikel in der Emulsion eine Größe im Bereich von 3 bis 500 nm aufweisen.

10. Verwendung nach einem der vorhergehenden Ansprüche, **dadurch gekennzeichnet, dass** das oder die aminierte(n) Silicon(e) der Formel (I) oder (II) in der Zusammensetzung für die Vorbehandlung in einem Mengenanteil von 0,01 bis 20 Gew.-% des Gesamtgewichts der Zusammensetzung enthalten sind.

11. Verwendung nach Anspruch 10, **dadurch gekennzeichnet, dass** das oder die aminierte(n) Silicon(e) der Formel (I) oder (II) in der Zusammensetzung für die Vorbehandlung in einem Mengenanteil von 0,1 bis 15 Gew.-% des Gesamtgewichts der Zusammensetzung enthalten sind.

12. Verwendung nach Anspruch 11, **dadurch gekennzeichnet, dass** das oder die aminierte(n) Silicon(e) der Formel (I) oder (II) in der Zusammensetzung für die Vorbehandlung in einem Mengenanteil von 0,5 bis 10 Gew.-% des Gesamtgewichts der Zusammensetzung enthalten sind.

13. Verwendung nach einem der vorhergehenden Ansprüche, **dadurch gekennzeichnet, dass** die Zusammensetzung für die Vorbehandlung als Lotion, Gel, Creme, Haarwaschmittel, Stift, Schaum oder Spray vorliegt.

14. Verwendung nach einem der vorhergehenden Ansprüche, **dadurch gekennzeichnet, dass** die Zusammensetzung für die Vorbehandlung in einem Pumpflakon oder einem Aerosolbehälter konfektioniert ist.

15. Verwendung nach Anspruch 14, **dadurch gekennzeichnet, dass** die Zusammensetzung für die Vorbehandlung mindestens ein Treibmittel enthält, das unter den Alkanen, Dimethylether, Stickstoff, Distickstoffoxid, Kohlendioxid oder Halogenalkanen ausgewählt ist.

16. Verwendung nach einem der vorhergehenden Ansprüche, **dadurch gekennzeichnet, dass** die Zusammensetzung für die Vorbehandlung mindestens einen grenzflächenaktiven Stoff vom nichtionischen, kationischen, anionischen oder amphoteren Typ enthält.

17. Verwendung nach Anspruch 16, **dadurch gekennzeichnet, dass** die Zusammensetzung für die Vorbehandlung ein Gemisch von grenzflächenaktiven Stoffen enthält, das mindestens einen anionischen grenzflächenaktiven Stoff umfasst, wobei der oder die anderen grenzflächenaktiven Stoffe nichtionisch oder amphoter sind.

18. Verwendung nach einem der vorhergehenden Ansprüche, **dadurch gekennzeichnet, dass** die Zusammensetzung für die Vorbehandlung mindestens ein ergänzendes Polymer enthält, das von den Siliconen der Formel (I) oder (II) verschieden ist.

19. Verwendung nach Anspruch 18, **dadurch gekennzeichnet, dass** das Polymer vom nichtionischen, kationischen, anionischen oder amphoteren Typ ist.

**20.** Verwendung nach Anspruch 19, **dadurch gekennzeichnet, dass** das Polymer ein aminiertes Silicon ist, das von den Siliconen der Formel (I) oder (II) verschieden ist.

**21.** Verwendung nach einem der vorhergehenden Ansprüche,
**dadurch gekennzeichnet, dass** die Zusammensetzung für die Vorbehandlung einen pH-Wert im Bereich von 2 bis 11 aufweist.

**22.** Verwendung nach Anspruch 1, um das Aufziehen der Färbung zu verbessern.

**23.** Verwendung nach Anspruch 22, um das Aufziehen der Färbung an strapazierten Haaren zu verbessern.

**24.** Verwendung nach Anspruch 23, um den Zustand der Fasern zu verbessern.

**25.** Verwendung nach Anspruch 24, um die Porosität der Fasern zu vermindern.

**26.** Verwendung nach Anspruch 24, um die alkalische Löslichkeit der Fasern zu vermindern.

**27.** Verwendung nach Anspruch 1, um die Beständigkeit der Färbung gegenüber Haarwäschen zu verbessern.

**28.** Verfahren zum Färben menschlicher Keratinfasern, **dadurch gekennzeichnet, dass** es darin besteht, in einem ersten Schritt eine Zusammensetzung für die Vorbehandlung, die mindestens ein Silicon der Formel (I) oder (II) enthält und wie sie in einem der Ansprüche 1 bis 9 beschrieben ist, auf die Fasern aufzutragen und dann, nachdem die Fasern gegebenenfalls gespült wurden, in einem zweiten Schritt eine Zusammensetzung für die Direktfärbung aufzutragen und während einer Zeitspanne einwirken zu lassen, die ausreichend ist, um die Farbe zu bilden, worauf die Fasern gegebenenfalls gespült und getrocknet werden.

**29.** Verfahren zum Färben menschlicher Keratinfasern, **dadurch gekennzeichnet, dass** es darin besteht, in einem ersten Schritt eine Zusammensetzung für die Vorbehandlung, die mindestens ein Silicon der Formel (I) oder (II) enthält und wie sie in einem der Ansprüche 1 bis 9 beschrieben ist, auf die Fasern aufzutragen und dann, nachdem die Fasern gegebenenfalls gespült wurden, in einem zweiten Schritt eine Zusammensetzung zum oxidativen Färben aufzutragen und während einer Zeitspanne einwirken zu lassen, die ausreichend ist, um die Farbe zu bilden, worauf die Fasern gegebenenfalls gespült und getrocknet werden.

**30.** Verfahren nach einem der Ansprüche 28 oder 29, **dadurch gekennzeichnet, dass** Zusammensetzung für die Vorbehandlung während einer Zeitspanne von einigen Sekunden bis 60 min einwirken gelassen wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 890335 A